# FASCICULE DE BREVET EUROPEEN

(11) **EP 1 357 887 B1**
(45) Date de publication et mention de la délivrance du brevet: **29.03.2006**
(21) Numéro de dépôt: 02700399.5
(22) Date de dépôt: 24.01.2002
(51) Int. Cl.: A61K 8/81, A61Q 5/04, A61Q 5/06

(54) **COMPOSITION COSMETIQUE COMPRENANT UN POLYMERE FIXANT ET UN POLY(VINYLLACTAME)CATIONIQUE**
KOSMETISCHE ZUSAMMENSETZUNG, DIE EIN FIXIERENDES POLYMER UND EIN KATIONISCHES POLYVINYLLACTAM ENTHÄLT
COSMETIC COMPOSITION COMPRISING A FIXING POLYMER AND A CATIONIC POLY(VINYLLACTAM)

(30) Priorité: 26.01.2001 FR 0101112
(43) Date de publication de la demande: 05.11.2003
(73) Titulaire: L'OREAL, 75008 Paris (FR)
(72) Inventeur: DE LA METTRIE, Roland, F-78110 Le Vésinet (FR); BELLI, Emanuelle, F-92600 Asnières (FR); MARIE, Laurence, F-93200 Saint-Denis (FR)
(74) Mandataire: Bourdeau, Françoise
(86) Numéro de dépôt international: PCT/FR2002/000292
(87) Numéro de publication internationale: WO 2002/058649

(56) Documents cités:
- EP-A- 0 219 830
- WO-A-00/68282
- FR-A- 2 203 831
- US-A- 5 523 369

## Description

L'invention a pour objet une composition cosmétique, notamment un gel ou une mousse, comprenant au moins un polymère fixant et au moins un polymère cationique particulier. Elle vise également un procédé cosmétique capillaire comprenant l'application de cette composition sur les cheveux ainsi que son utilisation pour fixer et/ou maintenir la coiffure.

Il est connu des produits de fixation et/ou de maintien de la coiffure se présentant sous diverses formes, notamment sous forme de gels ou de mousses. Si ceux-ci permettent souvent la fixation durable de la coiffure, ils présentent néanmoins divers inconvénients.

En particulier, de nombreux consommateurs reprochent que le gel reste collé sur les mains lors de l'application, que son temps de séchage sur les cheveux est long, ce qui peut entraîner un effondrement de la coiffure si le gel est appliqué en finition sur cheveux secs, et qu'il donne aux cheveux un aspect artificiel, en particulier une brillance non naturelle.

De même, lorsque la composition est conditionnée sous forme de mousse, les agents moussants peuvent donner à la composition un caractère collant au toucher. Par ailleurs, les cheveux ont parfois un aspect sec après application et séchage des mousses de coiffage.

Le problème posé par l'invention est de fournir une composition cosmétique capillaire, notamment sous forme de gel ou de mousse, qui soit améliorée par rapport aux compositions de l'art antérieur et, en particulier, qui laisse les mains propres après application sur les cheveux, donne lieu à une application sur des cheveux secs sans déstructuration la coiffure, sèche rapidement, maintienne fortement la forme de la coiffure et se répartisse de manière homogène, tout en donnant aux cheveux de bonnes propriétés cosmétiques.

De manière surprenante et inattendue, la Demanderesse a découvert qu'en sélectionnant judicieusement les constituants pour réaliser la composition cosmétique capillaire, il est possible de résoudre le problème posé ci-dessus.

L'invention a pour objet une composition cosmétique, caractérisée par le fait qu'elle comprend, dans un milieu cosmétiquement acceptable:
(i) au moins un polymère fixant choisi parmi les polymères fixants anioniques, amphotères, non ioniques et leurs mélanges et,
(ii) au moins un polymère poly(vinyllactame) cationique comprenant :
   -a) au moins un monomère de type vinyl lactame ou alkylvinyllactame;
   -b) au moins un monomère de structures (I) ou (II) suivantes : 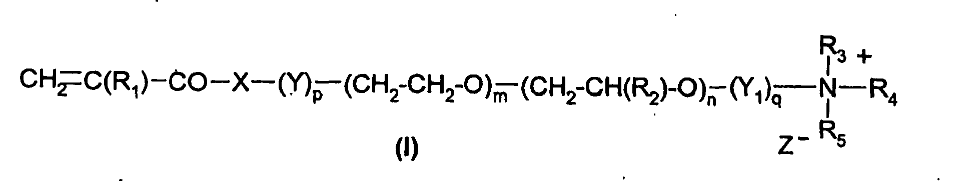 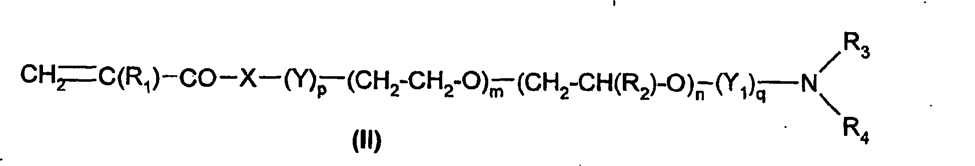
dans lesquelles :
X désigne un atome d'oxygène ou un radical NR₆,
R₁ et R₆ désignent, indépendamment l'un de l'autre, un atome d'hydrogène ou un radical alkyl linéaire ou ramifié en C₁-C₅,
R₂ désigne un radical alkyle linéaire ou ramifié en C₁-C₄,
R₃, R₄ et R₅ désignent, indépendamment l'un de l'autre, un atome d'hydrogène, un radical alkyle linéaire ou ramifié en C₁-C₃₀ ou un radical de formule (III) :

   ―(Y₂)ᵣ―(CH₂-CH(R₇)-O)ₓ―R₈ (III)
Y , Y₁ et Y₂ désignent, indépendamment l'un de l'autre, un radical alkylène linéaire ou ramifié en C₂-C₁₆,
R₇ désigne un atome d'hydrogène, ou un radical alkyle linéaire ou ramifié en C₁-C₄ ou un radical hydroxyalkyle linéaire ou ramifié en C₁-C₄,
R₈ désigne un atome d'hydrogène ou un radical alkyle linéaire ou ramifié en C₁-C₃₀,
p, q et r désignent, indépendamment l'un de l'autre, soit la valeur zéro, soit la valeur 1,
m et n désignent, indépendamment l'un de l'autre, un nombre entier allant de 0 à 100,
x désigne un nombre entier allant de 1 à 100,
Z désigne un anion d'acide organique ou minéral,
sous réserve que :
- l'un au moins des substituants R₃, R₄, R₅ ou R₈ désigne un radical alkyle linéaire ou ramifié en C₉-C₃₀,
- si m ou n est différent de zéro, alors q est égal à 1,
- si m ou n sont égaux à zéro, alors p ou q est égal à 0.

Un autre objet de l'invention concerne un procédé cosmétique capillaire, en particulier un procédé de fixation et/ou de maintien de la coiffure mettant en oeuvre ladite composition.

Un autre objet de l'invention concerne l'utilisation de cette composition pour le maintien et/ou à la mise en forme de la coiffure.

Sans vouloir être lié par une quelconque théorie, il semblerait que les avantages apportés par les poly(vinyllactame) cationiques selon la présente invention et tels que définis ci-après soient en relation avec un comportement de polymères épaississants de type associatif.

Les polymères associatifs sont des polymères dont les molécules sont capables, dans le milieu de formulation, de s'associer entre elles ou avec des molécules d'autres composés.

Leur structure chimique comprend généralement au moins une zone hydrophile et au moins une zone hydrophobe, la ou les zones hydrophobes comprenant au moins une chaîne grasse.

### Polymères polyvinyllactames cationiques selon l'invention

Les polymères poly(vinyllactame) cationiques selon l'invention comprennent :
-a) au moins un monomère de type vinyl lactame ou alkylvinyllactame;
-b) au moins un monomère de structures (I) ou (II) suivantes : 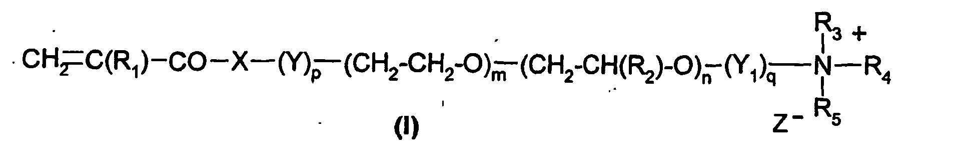 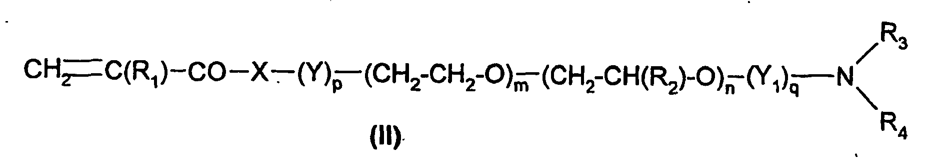 dans lesquelles :
   X désigne un atome d'oxygène ou un radical NR₆,
   R₁ et R₆ désignent, indépendamment l'un de l'autre, un atome d'hydrogène ou un radical alkyl linéaire du ramifié en C₁-C₅,
   R₂ désigne un radical alkyle linéaire ou ramifié en C₁-C₄,
   R₃, R₄ et R₅ désignent, indépendamment l'un de l'autre, un atome d'hydrogène, un radical alkyle linéaire ou ramifié en C₁-C₃₀ ou un radical de formule (III) :

      ―(Y₂)ᵣ―(CH₂-CH(R₇)-O)ₓ―R₈ (III)
   Y , Y₁ et Y₂ désignent, indépendamment l'un de l'autre, un radical alkylène linéaire ou ramifié en C₂-C₁₆,
   R₇ désigne un atome d'hydrogène, ou un radical alkyle linéaire ou ramifié en C₁-C₄ ou un radical hydroxyalkyle linéaire ou ramifié en C₁-C₄,
   Rg désigne un atome d'hydrogène ou un radical alkyle linéaire ou ramifié en C₁-C₃₀,
   p, q et r désignent, indépendamment l'un de l'autre, soit la valeur zéro, soit la valeur 1,
   m et n désignent, indépendamment l'un de l'autre, un nombre entier allant de 0 à 100,
   x désigne un nombre, entier allant de 1 à 100,
   Z désigne un anion d'acide organique ou minéral,
sous réserve que :
- l'un au moins des substituants R₃, R₄, R₅ ou R₈ désigne un radical alkyle linéaire ou ramifié en C₉-C₃₀,
- si m ou n est différent de zéro, alors q est égal à 1,
- si m ou n sont égaux à zéro, alors p ou q est égal à 0.

Les polymères poly(vinyllactame) cationiques selon l'invention peuvent être réticulés ou non réticulés et peuvent aussi être des polymères blocs.

De préférence le contre ion Z⁻ des monomères de formule (I) est choisi parmi les ions halogénures, les ions phosphates, l'ion méthosulfate, l'ion tosylate.

De préférence R₃, R₄ et R₅ désignent, indépendamment l'un de l'autre, un atome d'hydrogène ou un radical alkyle linéaire ou ramifié en C₁-C₃₀.
Plus préférentiellement, le monomère b) est un monomère de formule (I) pour laquelle, encore plus préférentiellement, m et n sont égaux à zéro.

Le monomère vinyl lactame ou alkylvinyllactame est de préférence un composé de structure (IV) : 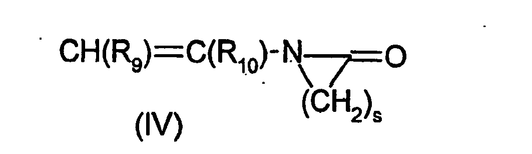 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

Encore plus préférentiellement, le monomère (IV) est la vinylpyrrolidone.

Les polymères poly(vinyllactame) cationiques selon l'invention peuvent également contenir un ou plusieurs monomères supplémentaires, de préférence cationiques ou non ioniques.

A titre de composés plus particulièrement préférés selon l'invention, on peut citer les terpolymères suivants comprenant au moins :
a)-un monomère de formule (IV),
b)-un monomère de formule (I) dans laquelle p=1, q=0, R₃ et R₄ désignent, indépendamment l'un de l'autre, un atome d'hydrogène ou un radical alkyle en C₁-C₅ et R₅ désigne un radical alkyle en C₉-C₂₄ et
c)-un monomère de formule (II) dans laquelle R₃ et R₄ désignent, indépendamment l'un de l'autre, un atome d'hydrogène ou un radical alkyle en C₁-C₅.

Encore plus préférentiellement, on utilisera les terpolymères comprenant, en poids, 40 à 95% de monomère (a), 0,25 à 50% de monomère (b) et 0,1 à 55% de monomère (c).
De tels polymères sont décrits dans la demande de brevet WO-00/68282

Comme polymères poly(vinyllactame) cationiques selon l'invention, on utilise notamment les terpolymères vinylpyrrolidone / diméthylaminopropylméthacrylamide /tosylate de dodécyldiméthylméthacrylamidopropylammonium, les terpolymères vinylpyrrolidone /diméthylaminopropylméthacrylamide/ tosylate de cocoyldiméthylméthacrylamidopropylammonium, les terpolymères vinylpyrrolidone /diméthylaminopropylméthacrylamide / tosylate ou chlorure de lauryldiméthylméthacrylamidopropylammonium.

La masse moléculaire en poids des polymères poly(vinyllactame) cationiques selon la présente invention est de préférence comprise entre 500 et 20 000 000. Elle est plus particulièrement comprise entre 200 000 et 2 000 000 et encore plus préférentiellement comprise entre 400 000 et 800 000.

Les polymères poly(vinyllactame) cationiques conformes à l'invention sont présents dans les compositions dans des concentrations allant de 0,01 à 30% en poids, plus préférentiellement de 0,1 à 10%, et plus particulièrement encore de 0,5 à 2% en poids.

### POLYMERES FIXANTS

Le polymère fixant (b) est choisi parmi lès polymères fixants anionique, amphotère, non ionique et leurs mélanges. Un polymère fixant est un polymère capable de maintenir et/ou de fixer la forme de la coiffure.

Ces polymères fixants peuvent être utilisés sous forme solubilisée ou encore sous forme de dispersion de particules solides de polymère.

Les polymères fixants (b) anioniques, amphotères et non ioniques utilisables conformément à l'invention sont décrits ci-après.

Les polymères fixants anioniques généralement utilisés sont des polymères comportant des groupements dérivés d'acide carboxylique, sulfonique ou phosphorique et ont un poids moléculaire compris entre environ 500 et 5.000.000.

1) Les groupements carboxyliques sont apportés par des monomères mono ou diacides carboxyliques insaturés tels que ceux répondant à la formule : 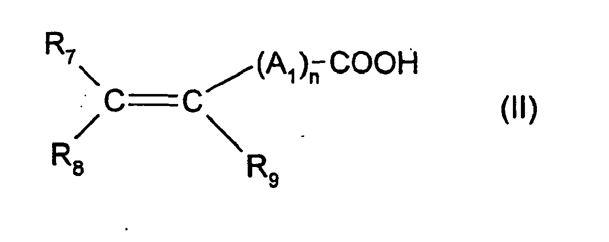 dans laquelle n est un nombre entier de 0 à 10, A₁ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ;
Dans la formule précitée un radical alkyle inférieur désigne de préférence un groupement ayant 1 à 4 atomes de carbone et en particulier, méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL E ou K par la société ALLIED COLLOID et ULTRAHOLD par la société BASF. Les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀ par exemple de lauryle tel que celui vendu par la société ISP sous la dénomination ACRYLIDONE LM et les terpolymères acide méthacrylique/ acrylate d'éthyle/ acrylate de tertiobutyle tel que le produit vendu sous la dénomination LUVIMER 100 P par la société BASF.
C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
D) les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. ; De tels polymères sont décrits en particulier dans les brevets US 2.047.398, 2.723.248, 2.102.113, le brevet GB 839.805 et notamment ceux vendus sous les dénominations GANTREZ AN ou ES par la société ISP.
   - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées.
   Ces polymères sont par exemple décrits dans les brevets français 2.350.384 et 2.357.241 de la demanderesse.
E) les polyacrylamides comportant des groupements carboxylates.

Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi :
- les sels de l'acide polyvinylsulfonique ayant un poids moléculaire compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire d'environ 500.000 et d'environ 100.000 vendus respectivement sous les dénominations Flexan 500 et Flexan 130 par National Starch. Ces composés sont décrits dans le brevet FR 2.198.719.
- les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.

Selon l'invention, les polymères fixants anioniques sont de préférence choisis parmi les copolymères d'acide acrylique tels que le terpolymère acide acrylique /acrylate d'éthyle / N-tertiobutylacrylamide vendu sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique / acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique mono estérifié vendu sous la dénomination GANTREZ ES 425 par la société ISP, les copolymères d'acide méthacrylique et de méthacrylate de méthyle vendus sous la dénomination EUDRAGIT L par la société ROHM PHARMA, le copolymère d'acide méthacrylique et d'acrylate d'éthyle vendu sous la dénomination LUVIMER MAEX ou MAE par la société BASF, le copolymère acétate de vinyle/acide crotonique vendu sous la dénomination LUVISET CA 66 par la société BASF et le copolymère acétate de vinyle/acide crotonique greffé par du polyéthylèneglycol sous la dénomination ARISTOFLEX A par la société BASF.

Les polymères fixants anioniques les plus particulièrement préférés sont choisis parmi le copolymère méthylvinyléther / anhydride maléique mono estérifié vendu sous la dénomination GANTREZ ES 425 par la société ISP, le terpolymère acide acrylique /acrylate d'éthyle / N-tertiobutylacrylamide vendu sous la dénomination ULTRAHOLD STRONG par la société BASF, les copolymères d'acide méthacrylique et de méthacrylate de méthyle vendus sous la dénomination EUDRAGIT L par la société ROHM PHARMA, les terpolymères acétate de vinyle / tertio-butyl benzoate de vinyle /acide crotonique et les terpolymères acide crotonique / acétate de vinyle /néododécanoate de vinyle vendus sous la dénomination Résine 28-29-30 par la société NATIONAL STARCH, le copolymère d'acide méthacrylique et d'acrylate d'éthyle vendu sous la dénomination LUVIMER MAEX OU MAE par la société BASF, le terpolymère de vinylpyrrolidone / acide acrylique/méthacrylate de lauryle vendu sous la dénomination ACRYLIDONE LM par la société ISP.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylamino-alkylméthacrylate et acrylate, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) les polyamino amides réticulés et alcoylés partiellement ou totalement dérivant de polyaminoamides de formule générale III: 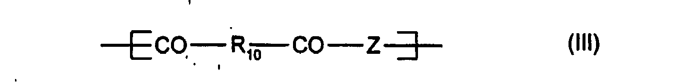 dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical IV 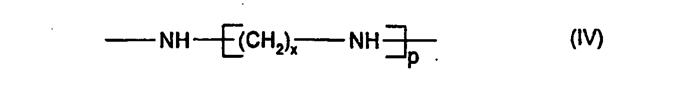 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (IV) ci-dessus, dans lequel x=2 et p=1 et qui dérive de l'éthylènediamine, ou le radical dérivant de la pipérazine : 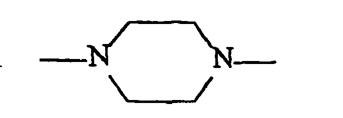
   c) dans les proportions de 0 à 20 moles % le radical -NH-(CH₂)₆-NH-dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) les polymères comportant des motifs zwittérioniques de formule V: 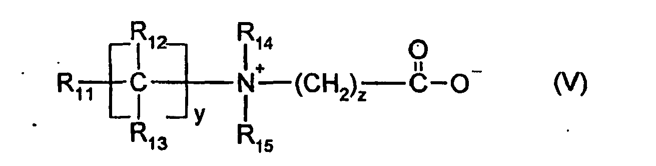 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentent un atome d'hydrogène, méthyle, éthyle ou propyle, R₁₄ et R₁₅ représentent un atome d'hydrogène ou un radical alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle /diméthyl carboxyméthylammonio éthyl tel que le produit vendu sous la dénomination DIAFORMER Z301 par la société SANDOZ.
(5) les polymères dérivés du chitosane comportant des motifs monomères répondant aux formules suivantes : 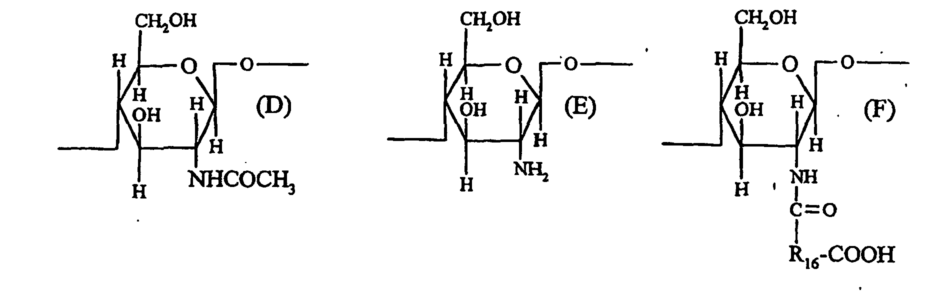 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 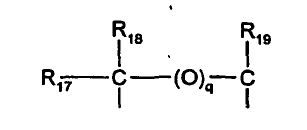 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcolylthio, sulfonique, un reste àlcoylthio dont le groupe alcoyle porte un reste amino, l'un au moins des radicaux R₁₇, R₁₈ et R₁₉ étant dans ce cas un atome d'hydrôgène ;
   ou si q=1, R₁₇, R₁₈ et R₁₉ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (VI) par exemple décrits dans le brevet français 1 400 366 : 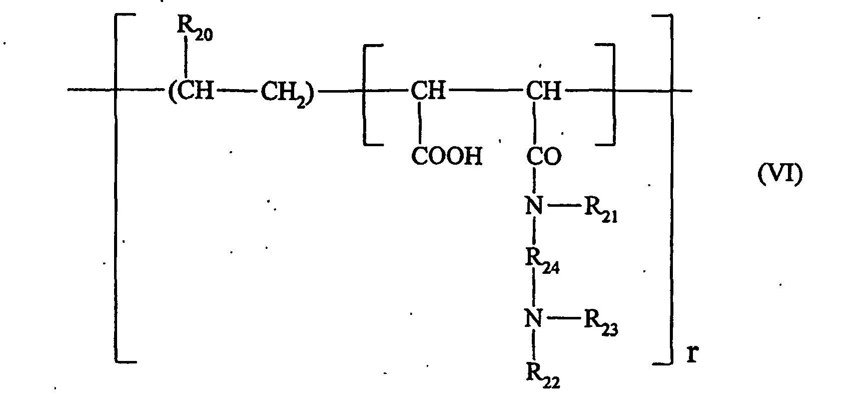 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂- , -CH₂-CH₂-CH₂- , -CH₂-CH(CH₃)- , R₂₂ ayant les significations mentionnées ci-dessus,
   ainsi que les homologues supérieurs de ces radicaux et contenant jusqu'à 6 atomes de carbone. '
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (VII)

      où D désigne un radical 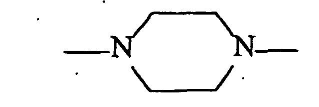 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
   b) Les polymères de formule :

      -D-X-D-X- (VII')

      où D désigne un radical 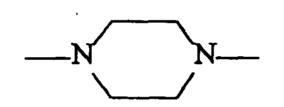 et X désigne le symbole E ou E' et au moins une fois E ; E ayant la signification indiquée ci-dessus et E' est un radical bivalent qui est un radical alkylène à chaîne droite
      ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C1-C5)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères fixants amphotères particulièrement préférés selon l'invention sont ceux de la famille (3) tels que les copolymères dont la dénomination CTFA est Octylacrylamide/acrylates/butylaminoethylmethacrylate copolymer tels que les produits vendus sous les dénominations AMPHOMER, AMHOMER LV 71 ou LOVOCRYL 47 par la société NATIONAL STARCH et ceux de la famille (4) tels que les copolymère de méthacrylate de butyle / méthacrylate de diméthyl carboxyméthylammonio- éthyl par exemple vendu sous la dénomination DIAFORMER Z301 par la société SANDOZ.

Les polymères fixants non ioniques utilisables selon la présente invention sont choisis par exemple parmi :
- les homopolymères de vinylpyrrolidone ;
- les copolymères de vinylpyrrolidone et d'acétate de vinyle ;
- les polyalkyloxazolines telles que les polyéthyloxazolines proposées par la société DOW CHEMICAL sous les dénominations PEOX 50 000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST ;
- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213
ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.

Les radicaux alkyle des polymères non ioniques ont de 1 à 6 atomes de carbone sauf mention contraire.

Les polymères non ioniques qui conviennent tout particulièrement pour la réalisation des compositions conformés à l'invention sont ceux choisis parmi:
* les copolymères de vinyllactame tels que les copolymères de vinylpyrrolidone et d'acétate de vinyle et les copolymères vinylpyrrolidone/acétate de vinyle/ propionate de vinyle
* la,polyvinylcaprolactame LUVISKOL PLUS (BASF)
* les homopolymères d'acétate de vinyle tels que APPRETAN EM (HOECHST) ou RHODOPAS A 012 (RHONE POULENC)
* les polyalkyloxazolines tels que PEOX 50 000 et PEOX 500 000 (DOW CHEMICAL)
* les copolymères d'acétate de vinyle et d'ester acrylique tels que le RHODOPAS AD 310 (RHONE POULENC)
* les copolymères d'acétate de vinyle et d'éthylène tels que APPRETAN TV (HOECHST)
* les copolymères d'acétate de vinyle et d'ester maléique tels que APPRETAN MB EXTRA (HOECHST)
* les homopolymères d'acrylates d'alkyle et les homopolymères de métacrylates d'alkyle tels que LUHYDRAN A 848 S (BASF)
* les copolymères d'esters acryliques tels que PRIMAL AC-261 K (ROHM & HAAS), ACRONAL 601 (BASF) ou APPRETAN N 9213 (HOECHST)
* les copolymères d'acrylonitrile et d'un monomère non-ionique tels que CJ 0601 B (ROHM & HAAS)
* les polyuréthanes tels que ACRYSOL RM 1020 ou ACRYSOL RM 2020 (ROHM & HAAS)
* les copolymères d'acrylate d'alkyle et d'uréthane tels que 8538-33 (NATIONAL STARCH)
* les polyamides tels que ESTAPOR LO 11 (RHONE POULENC)

Selon l'invention, on peut également utiliser les polymères fixants de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 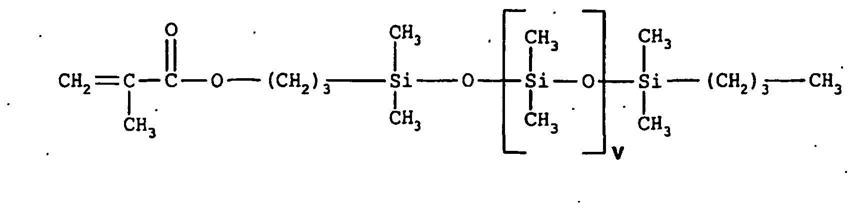
avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type pôly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut aussi utiliser, comme polymères fixants, des polyuréthannes fonctionnalisés ou non, siliconés ou non. A titre de polyuréthanne utilisable dans le cadre de l'invention, on peut citer le produit commercialisé sous l'appellation LUVISET PUR par BASF.

Les polyuréthannes particulièrement visés par la présente invention sont ceux décrits dans les brevets EP 0 751 162, EP 0 637 600, FR 2 743 297 et EP 0 648 485 dont la Demanderesse est Titulaire, ainsi que le brevets EP 0 656 021 ou WO 94/03510 de la Société BASF et EP 0 619 111 de la Société National Starch.

Avantageusement, le polymère fixant est présent dans la composition conforme à l'invention à une concentration relative en poids comprise entre 0,1 et 10 %, de préférence comprise entre 0,5 et 5 %.

Le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en C₁-C₄.

Parmi ces alcools, on peut citer l'éthanol, l'isopropanol. L'éthanol est particulièrement préféré.

La composition de l'invention peut également contenir au moins un additif choisi parmi les tensioactifs anioniques, cationiques, non ioniques ou amphotères, les parfums, les filtres, les conservateurs, les protéines, les vitamines, les provitamines, les polymères autres que ceux de l'invention, les huiles végétales, minérales ou synthétiques, les polyols comme les glycols ou la glycérine, les silicones, les alcools gras et tout autre additif classiquement utilisé dans les compositions cosmétiques.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas ou substantiellement pas, altérées par l'addition envisagée.

Les compositions conformes à l'inventions peuvent être appliquées sur la peau, les ongles, les lèvres, les cheveux, les sourcils et les cils.

Les compositions conformes à l'invention sont particulièrement adaptées pour des cheveux secs ou humides, en tant que produits de coiffage.

L'invention va être plus complètement illustrée à l'aide des exemples non limitatifs suivants.

Tous les pourcentages sont des pourcentages relatifs en poids par rapport au poids total de la composition et m.a. signifie matière active.

### EXEMPLES :

On réalise les compositions suivantes conformes à l'invention.

### Exemple 1 : mousse sans propulseur (flacon-pompe Airspray F2)

| | |
|---|---|
| Polymère 1 | 2.5 % |
| PVP/VA | 1 % |
| Glycérol | 0.5 % |
| Conservateurs, parfum | |
| Eau | qsp100% |

### Exemple 2 : mousse aérosol

| | |
|---|---|
| Polymère 1 | 2.5 % . |
| PVP | 1 % |
| Aérogaz 3,2 N (Atochem) | 6 % |
| Conservateurs, parfum | |
| Eau | qsp 100% |

### Exemple 3 : gel

| | |
|---|---|
| Polymère 1 | 2.5 % |
| PVP/VA | 0.5%. |
| Jaguar HP 105 | 1 % |
| Conservateurs, parfum | |
| Eau | qsp 100% |

Le polymère 1 est un terpolymère vinylpyrrolidone / diméthyl amino propylméthacrylamide/ chlorure de lauryldiméthylméthacrylamido ammonium proposé par la Société ISP sous la référence POLYMER ACP-1234.

## Revendications

1. Composition cosmétique **caractérisée par le fait qu'**elle comprend, dans un milieu cosmétiquement acceptable:
(i) au moins un polymère fixant choisi parmi les polymères fixants anioniques, amphotères, non ioniques et leurs mélanges et,
(ii) au moins un polymère poly(vinyllactame) cationique comprenant :
-a) au moins un monomère de type vinyl lactame ou alkylvinyllactame;
-b) au moins un monomère de structures (I) ou (II) suivantes : 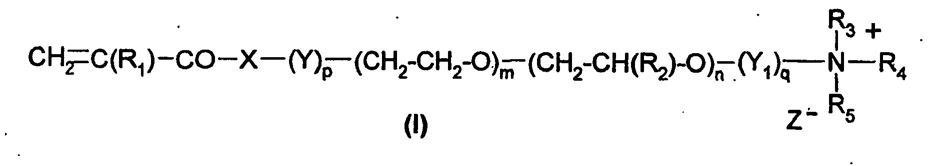 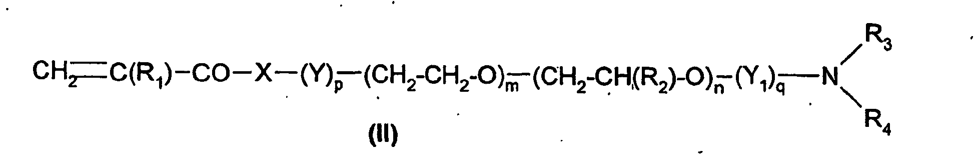
dans lesquelles :
X désigne un atome d'oxygène ou un radical NR₆,
R₁ et R₆ désignent, indépendamment l'un de l'autre, un atome d'hydrogène ou un radical alkyl linéaire ou ramifié en C₁-C₅,
R₂ désigne un radical alkyle linéaire ou ramifié en C₁-C₄,
R₃, R₄ et R₅ désignent, indépendamment l'un de l'autre, un atome d'hydrogène, un radical alkyle linéaire ou ramifié en C₁-C₃₀ ou un radical de formule (III) : 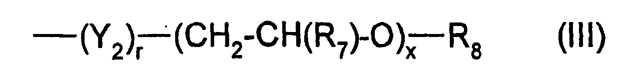
Y , Y₁ et Y₂ désignent, indépendamment l'un de l'autre, un radical alkylène linéaire ou ramifié en C₂-C₁₆,
R₇ désigne un atome d'hydrogène, ou un radical alkyle linéaire ou ramifié en C₁-C₄ ou un radical hydroxyalkyle linéaire ou ramifié en C₁-C₄,
R₈ désigne un atome d'hydrogène ou un radical alkyle linéaire ou ramifié en C₁-C₃₀,
p, q et r désignent, indépendamment l'un de l'autre, soit la valeur zéro, soit la valeur 1,
m et n désignent, indépendamment l'un de l'autre, un nombre entier allant de 0 à 100,
x désigne un nombre entier allant de 1 à 100,
Z désigne un anion d'acide organique ou minéral,
sous réserve que :
- l'un au moins des substituants R₃, R₄, R₅ ou R₈ désigne un radical alkyle linéaire ou ramifié en C₉-C₃₀,
- si m ou n est différent de zéro, alors q est égal à 1,
- si m ou n sont égaux à zéro, alors p ou q est égal à 0.

2. Composition selon la revendication 1 **caractérisée par le fait que** le monomère vinyl lactame ou alkylvinyllactame est un composé de structure (IV) : 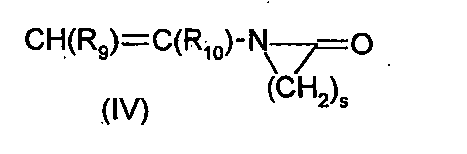 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

3. Composition selon la revendication 2 **caractérisée par le fait que** le monomère de formule (IV) est la vinylpyrrolidone.

4. Composition selon l'une quelconque des revendications précédentes, **caractérisée par le fait que** dans les formules (I) ou (II), les radicaux R₃, R₄, R₅ désignent, indépendamment l'un de l'autre, un atome d'hydrogène ou un radical alkyle linéaire ou ramifié en C₁-C₃₀.

5. Composition selon l'une quelconque des revendications précédentes, **caractérisée par le fait que** le monomère b) est un monomère de formule (I).

6. Composition selon la revendication 5, **caractérisée par le fait que** dans la formule (I), m et n sont égaux à zéro.

7. Composition selon l'une quelconque des revendications précédentes, **caractérisée par le fait que** le contre ion Z- des monomères de formule (I) est choisi parmi les ions halogénures, les ions phosphates, l'ion méthosulfate, l'ion tosylate.

8. Composition selon l'une quelconque des revendications 1 à 7, **caractérisée par le fait que** le ou les polymères poly(vinyllactame) cationiques contiennent un ou plusieurs monomères supplémentaires cationiques ou non ioniques.

9. Composition selon la revendication 8, **caractérisée par le fait que** le poly(vinyllactame) cationique est un terpolymère comprenant :
a)-un monomère de formule (IV),
b)-un monomère de formule (I) dans laquelle p=1, q=0, R₃ et R₄ désignent, indépendamment l'un de l'autre, un atome d'hydrogène ou un radical alkyle en C₁-C₅ et R₅ désigne un radical alkyle en C₉-C₂₄ et
c)-un monomère de formule (II) dans laquelle R₃ et R₄ désignent, indépendamment l'un de l'autre, un atome d'hydrogène ou un radical alkyle en C₁-C₅.

10. Composition selon la revendication 9, **caractérisée par le fait que** le terpolymère comprend en poids, 40 à 95% de monomère (a), 0,25 à 50% de monomère (b) et 0,1 à 55% de monomère (c).

11. Composition selon l'une quelconque des revendications précédentes, **caractérisée par le fait que** les poly(vinyllactame) cationiques sont choisis parmi les terpolymères vinylpyrrolidone / diméthylaminopropylméthacrylamide / tosylate de dodécyldiméthylméthacrylamidopropylammonium, les terpolymères vinylpyrrolidone /diméthylaminopropylméthacrylamide / tosylate de cocoyldiméthylméthacrylamidopropylammonium, les terpolymères vinylpyrrolidone /diméthylaminopropylméthacrylamide / tosylate ou chlorure de lauryldiméthylméthacrylamidopropylammonium.

12. Composition selon l'une quelconque des revendications précédentes, **caractérisée par le fait que** la masse moléculaire en poids des poly(vinyllactame) cationiques est comprise entre 500 et 20 000 000, de préférence comprise entre 200 000 et 2 000 000 et plus préférentiellement comprise entre 400 000 et 800 000. ,

13. Composition selon l'une quelconque des revendications précédentes, **caractérisée par** le fait le ou les poly(vinyllactame) cationiques sont utilisés en une quantité variant de 0,01 à 30% en poids du poids total de la composition.

14. Composition selon la revendication 13, **caractérisée par** le fait le ou les poly(vinyllactame) cationiques sont utilisés en une quantité variant de 0,1 à 10% en poids du poids total de la composition.

15. Composition selon l'une quelconque des revendication précédentes, **caractérisée par le fait que** le polymère fixant est un polymère anionique choisi parmi :
- les polymères comportant des motifs carboxyliques dérivant de monomères mono ou diacides carboxyliques insaturés de formule : 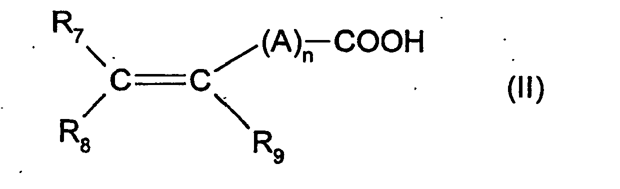
dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène; un groupement alkyle inférieur, un groupement -CH₂-COOH, phényle ou benzyle ;
- les polymères comprenant des motifs dérivant d'acide sulfonique tels que des motifs vinylsulfonique, styrènesulfonique, acrylamido alkylsulfonique.

16. Composition selon l'une quelconque des revendications 1 à 14, **caractérisée en ce que** le polymère fixant est un polymère amphotère choisi parmi les polymères comportant des motifs dérivant:
a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

17. Composition selon l'une quelconque des revendications 1 à 14, **caractérisée par le fait que** le polymère fixant est un polymère non ionique choisi parmi :
- les polyalkyloxazolines;
- les homopolymères d'acétate de vinyle;
- les copolymères d'acétate de vinyle et d'ester acrylique;
- les copolymères d'acétate de vinyle et d'éthylène;
- les copolymères d'acétate de vinyle et d'ester maléïque;
- les copolymères de polyéthylène et d'anhydride maléïque;
- les homopolymères d'acrylates d'alkyle et les homopolymères de méthacrylates d'alkyle;
- les copolymères d'esters acryliques tels que par exemple les copolymères d'acrylates d'alkyle et de méthacrylates d'alkyle ;
- les copolymères d'acrylonitrile et d'un monomère non ionique choisi par exemple parmi le butadiène et les (méth)acrylates d'alkyle;
- les copolymères d'acrylate d'alkyle et d'uréthanne ; et
- les polyamides.

18. Composition selon l'une quelconque des revendications 1 à 14, **caractérisée par le fait que** le polymère fixant est un polyuréthanne fonctionnalisé ou non, siliconé ou non.

19. Composition selon l'une quelconque des revendications 1 à 14, **caractérisée par le fait que** le polymère fixant est un polymère de type siliconé greffé comprenant une portion polysiloxane et une portion constituée d'une chaîne organique non-siliconée, l'une des deux portions constituant la chaîne principale du polymère l'autre étant greffée sur la dite chaîne principale.

20. Composition selon l'une quelconque des revendications précédentes, **caractérisée par le fait que** le polymère fixant est présent dans la composition à une concentration relative en poids comprise entre 0,1 et 10 %, de préférence comprise entre 0,5 et 5%.

21. Composition selon l'une quelconque des revendications précédentes, **caractérisée par le fait que** le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en C₁-C₄.

22. Composition selon l'une quelconque des revendications précédentes, **caractérisée par le fait qu'**elle contient au moins un additif choisi parmi les tensioactifs anioniques, cationiques, non ioniques ou amphotères, les parfums, les filtres, les conservateurs, les protéines, les vitamines, les provitamines, les polymères autres que ceux de l'invention, les huiles végétales, minérales ou synthétiques, les polyols comme les glycols ou là glycérine, les silicones, les alcools gras et tout autre additif classiquement utilisé dans les compositions cosmétiques.

23. Procédé cosmétique capillaire, **caractérisé par le fait qu'**il comprend l'application d'une composition selon l'une quelconque des revendications précédentes.

24. Utilisation d'une composition selon l'une quelconque des revendications 1 à 22. pour fixer et/ou mettre en forme la coiffure.

## Patentansprüche

1. Kosmetische Zusammensetzung, **dadurch gekennzeichnet, dass** sie in einem kosmetisch akzeptablen Medium enthält:
(i) mindestens ein fixierendes Polymer, das unter anionischen, amphoteren und nichtionischen fixierenden Polymeren und ihren Gemischen ausgewählt ist, und
(ii) mindestens ein kationisches Poly(vinyllactam)-Polymer, das enthält:
-a) mindestens ein Monomer vom Vinyllactam- oder Alkylvinyllactam-Typ;
-b) mindestens ein Monomer der nachstehenden Struktur (I) oder (II): 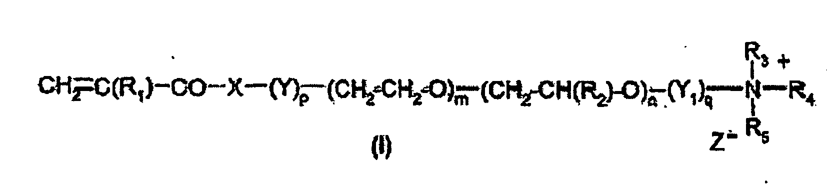 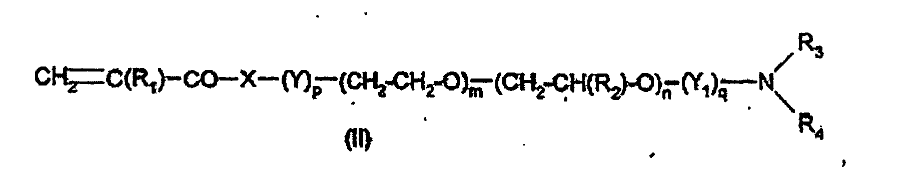
worin bedeuten:
X ein Wasserstoffatom oder eine Gruppe NR₆,
R₁ und R₆ unabhängig voneinander ein Wasserstoffatom oder
eine geradkettige oder verzweigte C₁₋₅-Alkylgruppe,
R₂ eine geradkettige oder verzweigte C₁₋₄-Alkylgruppe,
R₃, R₄ und R₅ unabhängig voneinander ein Wasserstoffatom, eine geradkettige oder verzweigte C₁₋₃-Alkylgruppe oder eine Gruppe der Formel (III): 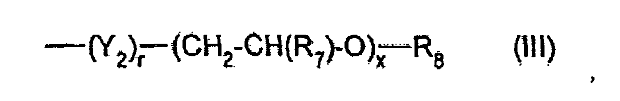
Y, Y₁ und Y₂ unabhängig voneinander eine geradkettige oder verzweigte C₂₋₁₆-Alkylgruppe,
R₇ ein Wasserstoffatom, eine geradkettige oder verzweigte C₁₋₄-Alkylgruppe oder eine geradkettige oder verzweigte C₁₋₄₋Hydroxyalkylgruppe,
R₈ ein Wasserstoffatom oder eine geradkettige oder verzweigte C₁₋₃₀- Alkylgruppe,
p, q und r unabhängig voneinander 0 oder 1,
m und n unabhängig voneinander eine ganze Zahl von 0 bis 100,
x eine ganze Zahl von 1 bis 100,
Z ein Anion einer organischen oder anorganischen Säure,
mit der Maßgabe, dass
- mindestens einer der Substituenten R₃, R₄, R₅ oder R₈ eine geradkettige oder verzweigte C₉₋₃₀-Alkylgruppe bezeichnet und,
- wenn m oder n von 0 verschieden ist, q gleich 1 ist,
- wenn m oder n gleich 0 ist, p oder q gleich 0 ist.

2. Zusammensetzung nach Anspruch 1, **dadurch gekennzeichnet, dass** das Vinyllactam-Monomer oder das Alkylvinyllactam-Monomer eine Verbindung der Struktur (IV) ist: 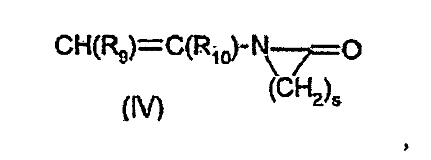 in der bedeuten:
s eine ganze Zahl von 3 bis 6,
R₉ ein Wasserstoffatom oder eine C₁₋₅-Alkylgruppe,
R₁₀ ein Wasserstoffatom oder eine C₁₋₅-Alkylgruppe,
mit der Maßgabe, dass mindestens einer der Substituenten R₉ und Rio ein Wasserstoffatom bedeutet.

3. Zusammensetzung nach Anspruch 2, **dadurch gekennzeichnet, dass** das Monomer der Formel (IV) Vinylpyrrolidon ist.

4. Zusammensetzung nach einem der vorhergehenden Ansprüche, **dadurch gekennzeichnet, dass** die Substituenten R₃, R₄, R₅ in Formel (I) oder (II) unabhängig voneinander ein Wasserstoffatom oder eine geradkettige oder verzweigte C₁₋₃₀-Alkylgruppe bedeuten.

5. Zusammensetzung nach einem der vorhergehenden Ansprüche, **dadurch gekennzeichnet, dass** das Monomer b) ein Monomer der Formel (I) ist.

6. Zusammensetzung nach Anspruch 5, **dadurch gekennzeichnet, dass** in Formel (I) m und n gleich Null sind.

7. Zusammensetzung nach einem der vorhergehenden Ansprüche, **dadurch gekennzeichnet, dass** das Gegenion Z- der Monomeren der Formel (I) unter Halogenidionen, Phosphationen, dem Methosulfat-Ion und dem Tosylat-Ion ausgewählt ist.

8. Zusammensetzung nach einem der Ansprüche 1 bis 7, **dadurch gekennzeichnet, dass** das oder die kationischen Poly(vinyllactam)-Polymeren ein oder mehrere zusätzliche kationische oder nichtionische Monomere enthalten.

9. Zusammensetzung nach Anspruch 8, **dadurch gekennzeichnet, dass** das kationische Polyvinyllactam ein Terpolymer ist, das enthält:
a) - ein Monomer der Formel (IV),
b) - ein Monomer der Formel (I), in der p=1 und q=0 sind und R₃ und R₄ unabhängig voneinander ein Wasserstoffatom oder eine C₁₋₅-Alkylgruppe bezeichnen und R₅ eine C₉₋₂₄₋Alkylgruppe bezeichnet, und
c) - ein Monomer der Formel (II), in der R₃ und R₄ unabhängig voneinander ein Wasserstoffatom oder eine C₁₋₅-Alkylgruppe bedeuten.

10. Zusammensetzung nach Anspruch 9, **dadurch gekennzeichnet, dass** das Terpolymer 40 bis 95 Gew.-% Monomer (a), 0,25 bis 50 Gew.-% Monomer (b) und 0,1 bis 55 Gew.-% Monomer (c) enthält.

11. Zusammensetzung nach einem der vorhergehenden Ansprüche, **dadurch gekennzeichnet, dass** die kationischen Poly(vinyllactame) ausgewählt sind unter Vinylpyrrolidon / Dimethylaminopropylmethacrylamid/ Dodecyldimethylmethacrylamidopropylammoniumtosylat-Terpolymeren, Vinylpyrrolidon / Dimethylaminopropylmethacrylamid / Cocoyldimethylmethacrylamidopropylammoniumtosylat-Terpolymeren, Vinylpyrrolidon/ Dimethylaminopropylmethacrylamid/Lauryldimethylmethacrylamidopropylammoniumtosylat oder -chlorid-Terpolymeren.

12. Zusammensetzung nach einem der vorhergehenden Ansprüche, **dadurch gekennzeichnet, dass** die gewichtsbezogene Molekülmasse der kationischen Polyvinyllactame im Bereich von 500 bis 20.000.000, bevorzugt im Bereich von 200.000 bis 2.000.000 und noch bevorzugter im Bereich von 400.000 bis 800.000 liegt.

13. Zusammensetzung nach einem der vorhergehenden Ansprüche, **dadurch gekennzeichnet, dass** das oder die kationischen Polyvinyllactame in einer Menge eingesetzt sind, die im Bereich von 0,01 bis 30 Gew.-%, bezogen auf das Gesamtgewicht der Zusammensetzung, varüert.

14. Zusammensetzung nach Anspruch 13, **dadurch gekennzeichnet, dass** das oder die kationischen Polyvinyllactame in einer Menge eingesetzt sind, die im Bereich von 0,1 bis 10 Gew.-%, bezogen auf das Gesamtgewicht der Zusammensetzung, variiert.

15. Zusammensetzung nach einem der vorhergehenden Ansprüche, **dadurch gekennzeichnet, dass** das fixierende Polymer ein anionisches Polymer ist, das ausgewählt ist unter:
- Polymeren, die Carboxylgruppen enthaltende Einheiten aufweisen, die von monomeren ungesättigten Mono- oder Dicarbonsäuren der Formel 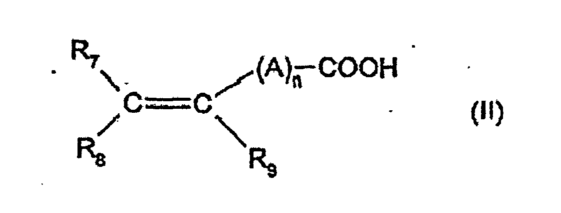 abgeleitet sind, worin bedeuten:
n eine ganze Zahl von 0 bis 10,
A eine Methylengruppe, die gegebenenfalls, wenn n größer als 1 ist, über ein Heteroatom wie Sauerstoff oder Schwefel an dem Kohlenstoffatom der ungesättigten Gruppe oder der benachbarten Methylengruppe gebunden ist,
R₇ ein Wasserstoffatom, eine Phenylgruppe oder eine Benzylgruppe,
R₈ ein Wasserstoffatom, eine niedere Alkylgruppe oder Carboxy,
R₉ ein Wasserstoffatom, eine niedere Alkylgruppe, eine Gruppe -CH₂-COOH, Phenyl oder Benzyl;
- Polymeren, die von einer Sulfonsäure abgeleitete Einheiten enthalten, wie Vinylsulfonsäure-Einheiten, Styrolsulfonsäure-Einheiten, Acrylamidoalkylsulfonsäure-Einheiten.

16. Zusammensetzung nach einem der Ansprüche 1 bis 14, **dadurch gekennzeichnet, dass** das fixierende Polymer ein amphoteres Polymer ist, das ausgewählt ist unter Polymeren, die Einheiten aufweisen, die abgeleitet sind von:
a) mindestens einem Monomer, das unter den Acrylamiden oder Methacrylamiden ausgewählt ist, die am Stickstoff mit einer Alkylgruppe substituiert sind,
b) mindestens einem sauren Comonomer, das eine oder mehrere reaktive Carboxylgruppen enthält, und
c) mindestens einem basischen Comonomer, wie Estern mit primären, sekundären, tertiären und quaternären Aminsubstituenten von Acrylsäure und Methacrylsäure und dem Quaternisierungsprodukt von Dimethylethylmethacrylat mit Dimethylsulfat oder Diethylsulfat.

17. Zusammensetzung nach einem der Ansprüche 1 bis 14, **dadurch gekennzeichnet, dass** das fixierende Polymer ein nichtionisches Polymer ist, das ausgewählt ist unter
- den Polyalkyloxazolinen;
- Vinylacetat-Homopolymeren;
- Vinylacetat-Acrylester-Copolymeren;
- Vinylacetat-Ethylen-Copolymeren;
- Vinylacetat-Maleinsäureester-Copolymeren;
- Polyethylen-Maleinsäureanhydrid-Copolymeren;
- Alkylacrylat-Homopolymeren und Alkylmethacrylat-Homopolymeren;
- Copolymeren von Acrylsäureestern, wie zum Beispiel Copolymeren von Alkylacrylaten und Alkylmethacrylaten;
- Copolymeren von Acrylnitril mit einem nichtionischen Monomer, das zum Beispiel unter Butadien und Alkyl(meth)acrylaten ausgewählt ist;
- Copolymeren von Alkylacrylaten und Urethanen und
- Polyamiden.

18. Zusammensetzung nach einem der Ansprüche 1 bis 14, **dadurch gekennzeichnet, dass** das fixierende Polymer ein gegebenenfalls funktionalisiertes Polyurethan ist, das gegebenenfalls siliconhaltig ist.

19. Zusammensetzung nach einem der Ansprüche 1 bis 14, **dadurch gekennzeichnet, dass** das fixierende Polymer ein Polymer vom Typ eines gepfropften Silicons ist, das einen Polysiloxan-Teil sowie einen Teil aufweist, der aus einer nicht aus Silicon bestehenden organischen Kette besteht, wobei einer der beiden Teile die Hauptkette des Polymers darstellt und der andere Teil auf diese Hauptkette aufgepfropft ist.

20. Zusammensetzung nach einem der vorhergehenden Ansprüche, **dadurch gekennzeichnet, dass** das fixierende Polymer in der Zusammensetzung in einer Konzentration von 0,1 bis 10 Gew.-% und bevorzugt von 0,5 bis 5 Gew.-% vorliegt.

21. Zusammensetzung nach einem der vorhergehenden Ansprüche, **dadurch gekennzeichnet, dass** das kosmetisch akzeptable Medium bevorzugt aus Wasser oder einem oder mehreren kosmetisch akzeptablen Lösungsmitteln, wie Alkoholen oder Alkohol-Lösungsmittel-Gemischen besteht, wobei diese Lösungsmittel bevorzugt C₁₋₄-Alkohole sind.

22. Zusammensetzung nach einem der vorhergehenden Ansprüche, **dadurch gekennzeichnet, dass** sie mindestens einen Zusatzstoff enthält, der ausgewählt ist unter anionischen, kationischen, nichtionischen oder amphoteren grenzflächenaktiven Mitteln, Parfums, Filtern, Konservierungsmitteln, Proteinen, Vitaminen, Provitaminen, Polymeren, die von denen der Erfindung verschieden sind, Pflanzenölen, Mineralölen oder synthetischen Ölen, Polyolen, wie Glykolen oder Glycerin, Siliconen, Fettalkoholen sowie sämtlichen anderen Zusatzstoffen, die herkömmlicherweise in kosmetischen Zusammensetzungen eingesetzt werden.

23. Haarkosmetisches Verfahren, **dadurch gekennzeichnet, dass** es das Aufbringen einer Zusammensetzung nach einem der vorhergehenden Ansprüche umfasst.

24. Verwendung einer Zusammensetzung nach einem der Ansprüche 1 bis 22 zur Fixierung und/oder Formgebung der Frisur.

## Claims

1. Cosmetic composition, **characterized in that** it comprises, in a cosmetically acceptable medium:
(i) at least one fixing polymer chosen from anionic, amphoteric and nonionic fixing polymers, and mixtures thereof, and
(ii) at least one cationic poly(vinyllactam) polymer comprising:
-a) at least one monomer of vinyllactam or alkylvinyllactam type;
-b) at least one monomer of structure (I) or (II) below: 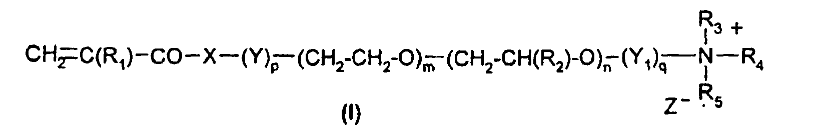 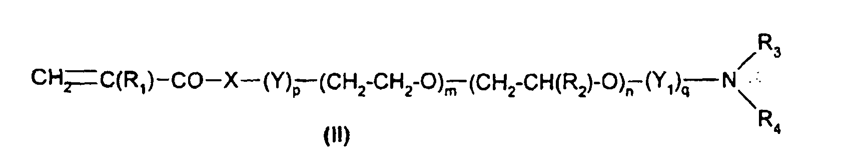
in which:
X denotes an oxygen atom or a radical NR₆,
R₁ and R₆ denote, independently of each other, a hydrogen atom or a linear or branched C₁-C₅ alkyl radical,
R₂ denotes a linear or branched C₁-C₄ alkyl radical,
R₃, R₄ and R₅ denote, independently of each other, a hydrogen atom, a linear or branched C₁-C₃₀ alkyl radical or a radical of formula (III):
-(Y₂)ᵣ-(CH₂-CH(R₇)-O)ₓ-R₈ (III)
Y, Y₁ and Y₂ denote, independently of each other, a linear or branched C₂-C₁₆ alkylene radical,
R₇ denotes a hydrogen atom or a linear or branched C₁-C₄ alkyl radical or a linear or branched C₁-C₄ hydroxyalkyl radical,
R₈ denotes a hydrogen atom or a linear or branched C₁-C₃₀ alkyl radical,
p, q and r denote, independently of each other, either the value 0 or the value 1,
m and n denote, independently of each other, an integer ranging from 0 to 100,
x denotes an integer ranging from 1 to 100,
Z denotes an organic or mineral acid anion,
with the proviso that:
- at least one of the substituents R₃, R₄, R₅ or R₈ denotes a linear or branched C₉-C₃₀ alkyl radical,
- if m or n is other than zero, then q is equal to 1,
- if m or n are equal to zero, then p or q is equal to 0.

2. Composition according to Claim 1,
**characterized in that** the vinyllactam or alkylvinyllactam monomer is a compound of structure (IV): 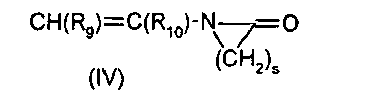 in which:
s denotes an integer ranging from 3 to 6,
R₉ denotes a hydrogen atom or a C₁-C₅ alkyl radical,
R₁₀ denotes a hydrogen atom or a C₁-C₅ alkyl radical,
with the proviso that at least one of the radicals R₉ and R₁₀ denotes a hydrogen atom.

3. Composition according to Claim 2,
**characterized in that** the monomer of formula (IV) is vinylpyrrolidone.

4. Composition according to any one of the preceding claims, **characterized in that**, in formula (I) or (II), the radicals R₃, R₄ and R₅ denote, independently of each other, a hydrogen atom or a linear or branched C₁-C₃₀ alkyl radical.

5. Composition according to any one of the preceding claims, **characterized in that** the monomer b) is a monomer of formula (I).

6. Composition according to Claim 5,
**characterized in that**, in formula (I), m and n are equal to zero.

7. Composition according to any one of the preceding claims, **characterized in that** the counterion Z⁻ of the monomers of formula (I) is chosen from halide ions, phosphate ions, the methosulphate ion and the tosylate ion.

8. Composition according to any one of Claims 1 to 7, **characterized in that** the cationic poly(vinyllactam) polymer(s) contain(s) one or more additional cationic or nonionic monomers.

9. Composition according to Claim 8,
**characterized in that** the cationic poly(vinyllactam) is a terpolymer comprising:
a) a monomer of formula (IV),
b) a monomer of formula (I) in which p=1, q=0, R₃ and R₄ denote, independently of each other, a hydrogen atom or a C₁-C₅ alkyl radical and R₅ denotes a C₉-C₂₄ alkyl radical, and
c) a monomer of formula (II) in which R₃ and R₄ denote, independently of each other, a hydrogen atom or a C₁-C₅ alkyl radical.

10. Composition according to Claim 9, **characterized in that** the terpolymer comprises, by weight, 40% to 95% of monomer (a), 0.25% to 50% of monomer (b) and 0.1% to 55% of monomer (c).

11. Composition according to any one of the preceding claims, **characterized in that** the cationic poly(vinyllactams) are chosen from vinylpyrrolidone/dimethylaminopropylmethacrylamide/dodecyldimethylmethacrylamidopropylammonium tosylate terpolymers, vinylpyrrolidone/dimethylaminopropylmethacrylamide/cocoyldimethylmethacrylamidopropylammonium tosylate terpolymers, vinylpyrrolidone/dimethylaminopropylmethacrylamide/lauryldimethylmethacrylamidopropylammonium tosylate or chloride terpolymers.

12. Composition according to any one of the preceding claims, **characterized in that** the weight-average molecular mass of the cationic poly(vinyllactams) is between 500 and 20 000 000, preferably between 200 000 and 2 000 000 and even more preferably between 400 000 and 800 000.

13. Composition according to any one of the preceding claims, **characterized in that** the cationic poly(vinyllactam(s)) is (are) used in an amount ranging from 0.01% to 30% by weight relative to the total weight of the composition.

14. Composition according to Claim 13, **characterized in that** the cationic poly(vinyllactam(s)) is (are) used in an amount ranging from 0.1% to 10% by weight relative to the total weight of the composition.

15. Composition according to any one of the preceding claims, **characterized in that** the fixing polymer is an anionic polymer chosen from:
- polymers comprising carboxylic units derived from unsaturated monocarboxylic or dicarboxylic acid monomers of formula: 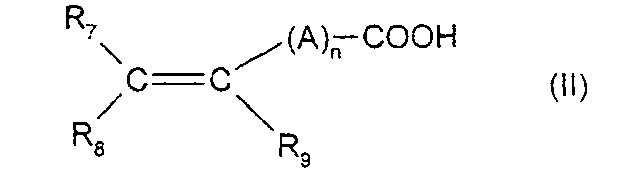 in which n is an integer from 0 to 10, A denotes a methylene group, optionally connected to the carbon atom of the unsaturated group, or to the neighboring methylene group when n is greater than 1, via a hetero atom such as oxygen or sulphur, R₇ denotes a hydrogen atom or a phenyl or benzyl group, R₈ denotes a hydrogen atom or a lower alkyl or carboxyl group, R₉ denotes a hydrogen atom, a lower alkyl group or a -CH₂-COOH, phenyl or benzyl group;
- polymers comprising units derived from sulphonic acid, such as vinylsulphonic, styrenesulphonic or acrylamidoalkylsulphonic units.

16. Composition according to any one of Claims 1 to 14, **characterized in that** the fixing polymer is an amphoteric polymer chosen from polymers comprising units derived from:
a) at least one monomer chosen from acrylamides and methacrylamides substituted on the nitrogen with an alkyl radical,
b) at least one acidic comonomer containing one or more reactive carboxylic groups, and
c) at least one basic comonomer such as esters containing primary, secondary, tertiary and quaternary amine substituents of acrylic and methacrylic acids and the product of quaternization of dimethylaminoethyl methacrylate with dimethyl or diethyl sulphate.

17. Composition according to any one of Claims 1 to 14, **characterized in that** the fixing polymer is a nonionic polymer chosen from:
- polyalkyloxazolines;
- vinyl acetate homopolymers;
- copolymers of vinyl acetate and of acrylic ester;
- copolymers of vinyl acetate and of ethylene;
- copolymers of vinyl acetate and of maleic ester;
- copolymers of polyethylene and of maleic anhydride;
- alkyl acrylate homopolymers and alkyl methacrylate homopolymers;
- acrylic ester copolymers such as, for example, copolymers of alkyl acrylates and of alkyl methacrylates;
- copolymers of acrylonitrile and of a nonionic monomer chosen, for example, from butadiene and alkyl (meth)acrylates;
- copolymers of alkyl acrylate and of urethane; and
- polyamides.

18. Composition according to any one of Claims 1 to 14, **characterized in that** the fixing polymer is a functionalized or nonfunctionalized, silicone or nonsilicone polyurethane.

19. Composition according to any one of Claims 1 to 14, **characterized in that** the fixing polymer is a polymer of grafted silicone type comprising a polysiloxane portion and a portion consisting of a nonsilicone organic chain, one of the two portions constituting the main chain of the polymer, the other being grafted onto said main chain.

20. Composition according to any one of the preceding claims, **characterized in that** the fixing polymer is present in the composition at a relative weight concentration of between 0.1% and 10% and preferably between 0.5% and 5%.

21. Composition according to any one of the preceding claims, **characterized in that** the cosmetically acceptable medium preferably consists of water or of one or more cosmetically acceptable solvents such as alcohols or water/solvent(s) mixtures, these solvents preferably being C₁-C₄ alcohols.

22. Composition according to any one of the preceding claims, **characterized in that** it contains at least one additive chosen from anionic, cationic, nonionic and amphoteric surfactants, fragrances, screening agents, preserving agents, proteins, vitamins, provitamins, polymers other than those of the invention, plant, mineral or synthetic oils, polyols, for instance glycols or glycerol, silicones, fatty alcohols and any other additive conventionally used in cosmetic compositions.

23. Cosmetic haircare process, **characterized in that** it comprises the application of a composition according to any one of the preceding claims.

24. Use of a composition according to any one of Claims 1 to 22 to fix and/or shape the hairstyle.
